# EUROPEAN PATENT APPLICATION

(11) **EP 4 772 188 A1**
(43) Date of publication of application: **08.07.2026**
(21) Application number: 25224953.7
(22) Date of filing: 18.12.2025
(51) Int. Cl.: A61K 36/8962, A61K 36/9066, A61K 31/122, A23L 33/15, A23L 33/155, A61P 9/00

(54) **ASSOCIATION OF ACTIVE INGREDIENTS FOR USE IN PREVENTING AND/OR TREATING THE DYSFUNCTION OF THE VASCULAR AND/OR VALVULAR ENDOTHELIUM OF THE CARDIOVASCULAR SYSTEM**

(30) Priority: 02.01.2025 IT 202500000006
(71) Applicant: Gricar Chemical S.r.l., 20861 Brugherio MB (IT); Universita' degli Studi di Milano, 20122 Milano (IT); Centro Cardiologico Monzino S.p.A., 20121 Milano (IT)
(72) Inventor: Carenzi, Gian Marco, 20861 Brugherio MB (IT); Pompilio, Giulio, 20121 Milano MI (IT); Poggio, Paolo, 20121 Milano MI (IT)
(74) Representative: Perani & Partners S.p.A.

(57) **Abstract**

The present invention relates to an association of active ingredients comprising: a dry extract of Curcuma, a dry extract of fermented black garlic, a coenzyme Q10, at least one vitamin selected from the group consisting of: at least one B-group vitamin; vitamin D; and combinations of the foregoing, for use in the treatment and/or prevention of the dysfunction of the vascular and/or valvular endothelium of the cardiocirculatory system.

## Description

### FIELD OF THE INVENTION

The present patent application relates to an association of active ingredients directed to restoring and/or maintaining the physiological function of the vascular and/or valvular endothelium in the context of the cardiocirculatory system.

### STATE OF THE ART

Aortic valve sclerosis (AVSc) represents a pathological condition of growing clinical and scientific interest, given its prevalence (over 30% in adults over 65 years of age) and its prognostic implications [1-4]. The understanding of pathogenic mechanisms shared with coronary artery disease (CAD) has opened new prospects for research and treatment [2]. Endothelial dysfunction, which represents the first step towards many cardiovascular pathologies, is a key element in both conditions [5, 6].

The increase in oxidative stress, due to an imbalance between the production of reactive oxygen species and antioxidant defense mechanisms, plays a crucial role in endothelial damage and valvular calcification [7, 8]. The chronic infiltration of inflammatory cells and the deposition of lipoproteins in the aortic valve contribute to the progression of sclerosis, similarly to what occurs in the coronary arteries [7, 8]. Epidemiological studies have shown that traditional risk factors for CAD, such as advanced age, hypertension, dyslipidemia, and diabetes, are also strongly associated with AVSc [2, 3]. The latter is also strictly linked to the development of aortic stenosis (AS), which occurs in 16% of subjects with AVSc [9]. All this suggests that interventions aimed at controlling these risk factors could have a significant impact on the prevention and management of AVSc and the potential subsequent AS. Finally, the concomitant presence of CAD and AVSc in patients represents a significant clinical challenge, as both conditions are associated with an increase in short- and long-term mortality [2, 3]. Therefore, an integrated and multidisciplinary management of these patients is essential for improving clinical outcomes.

Endothelial cells, which line the inside of blood vessels and the surface of heart valves, play a crucial role in maintaining vascular and valvular homeostasis through a series of complex and interconnected processes [10]. These cells act as a selective barrier that regulates the passage of substances and cells between the blood and surrounding tissues. Among the key processes are the regulation of vascular tone, the modulation of the inflammatory response, the prevention of thrombosis, and the promotion of tissue repair [11].

In particular, endothelial cells produce and release a variety of bioactive molecules, such as nitric oxide (NO), which plays a fundamental role in vasodilation and protection against atherosclerosis [11]. Moreover, these cells are involved in regulating the balance between coagulation and fibrinolysis, preventing the formation of clots that could obstruct blood vessels [12]. The ability of endothelial cells to respond to mechanical and chemical stimuli is essential for adapting to hemodynamic variations and maintaining the structural and functional integrity of the cardiovascular system [12]. Therefore, endothelial dysfunction, characterized by a reduced production of NO and an increase in oxidative stress, represents a critical factor in the development of many cardiovascular pathologies, including aortic valve sclerosis and coronary artery disease.

### Problem of the known art

In light of the aforementioned medical needs, there is a need to define an association of active ingredients capable of acting on the pathological condition of vascular and/or valvular endothelial dysfunction and/or maintaining endothelial homeostasis under physiological conditions.

### SUMMARY OF THE INVENTION

The Applicant has developed an association of active ingredients comprising
- dry extract of Curcuma,
- dry extract of fermented black garlic,
- coenzyme Q10,
- at least one vitamin selected from the group consisting of: at least one B-group vitamin; vitamin D; and combinations of the foregoing,
for use in the treatment and/or prevention of the dysfunction of the vascular and/or valvular endothelium of the cardiocirculatory system.

A second object of the invention is an oral formulation comprising the aforementioned association of active ingredients in combination with suitable excipients and/or diluents.

### Advantages of the invention

The association of the invention is advantageous because it effectively allows for maintaining the physiological function of the vascular and/or valvular endothelium and/or treating its dysfunction in case of pathological or altered conditions compared to the physiological condition.

As is evident from the Examples section, the association possesses anti-atherosclerotic, anti-oxidative stress and/or anti-inflammatory effects.

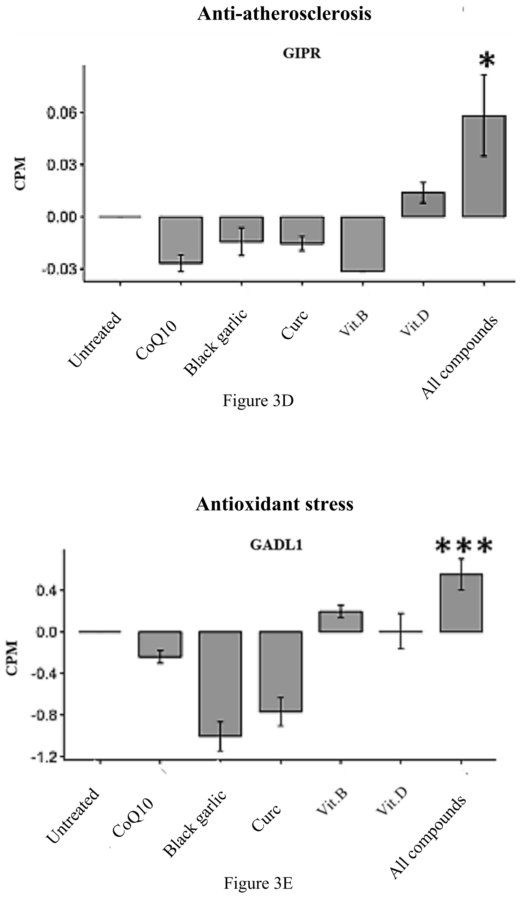

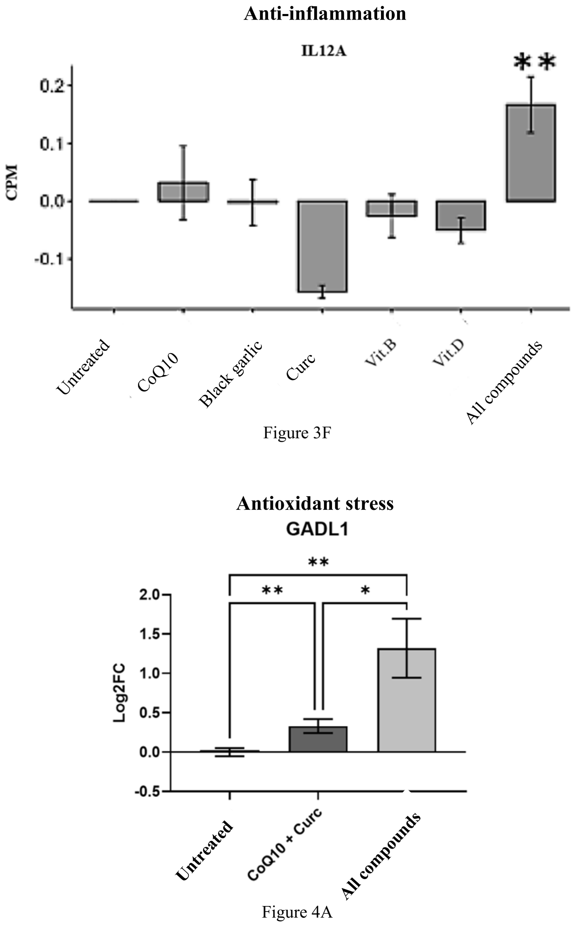

The Applicant has also demonstrated the synergy of the active ingredients comprised in the association in relation to three genes known to have anti-atherosclerotic (Gastric Inhibitory Polypeptide Receptor; GIPR), anti-oxidative stress (Glutamate Decarboxylase Like 1; GADL1) and anti-inflammatory (Interleukin 12A; IL12A) effects (see Example 2.3 and Figures 3D, 3E and 3F). It should be noted that some of the active ingredients, such as, for example, the dry extract of Curcuma, when taken individually, show an effect on GIPR, GADL1 and IL12A opposite to that of the association of active ingredients. This means that, individually, the compound has a gene-suppressing effect, which could be potentially negative for cellular homeostasis. However, in combination with the other compounds, the effect is to enhance the expression of these genes, resulting in a beneficial effect on valvular endothelial cells. The Applicant has also demonstrated that the association of the five compounds (black garlic, curcuma, coenzyme Q10, vitamin B1 and vitamin D3) determines an effect of contrasting inflammatory/oxidative mechanisms that is statistically superior to the effect induced by the "coenzyme Q10-curcuma" pair. In this way, the Applicant believes to have demonstrated that it is possible to achieve a synergistic effect among all the compounds comprised in the association, also with respect to the aforementioned pair of active ingredients.

The Applicant has, therefore, identified an association capable of effectively counteracting the mechanisms underlying the dysfunction of the vascular and/or valvular endothelium of the cardiocirculatory system, i.e., the inflammatory and oxidative stress mechanisms, which could trigger atherosclerotic processes.

### DESCRIPTION OF THE FIGURES

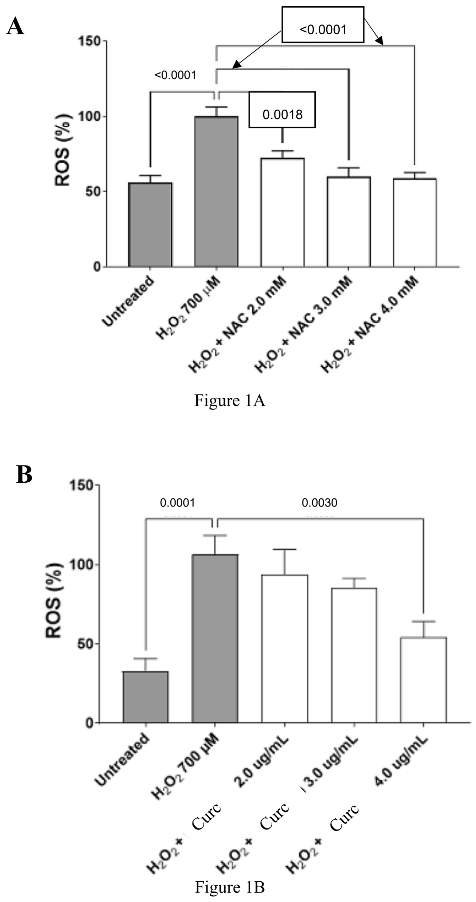

Figure 1A - Bar chart showing the percentage of ROS in the absence of treatment (first column from the left, untreated), after treatment with 700 µM H₂O₂ (second column from the left) and following the combination of 700 µM H₂O₂ with NAC at 2, 3 and 4 mM (white columns). The numbers indicated at the solid lines relate to the statistical data.
Figure 1B - Bar chart showing the percentage of ROS in the absence of treatment (first column from the left, untreated), after treatment with 700 µM H₂O₂ (second column from the left) and following the combination of 700 µM H₂O₂ with the starting material comprising Curcumin (indicated as "Curc") at 2, 3 and 4 µg/mL (white columns). The numbers indicated at the solid lines relate to the statistical data.

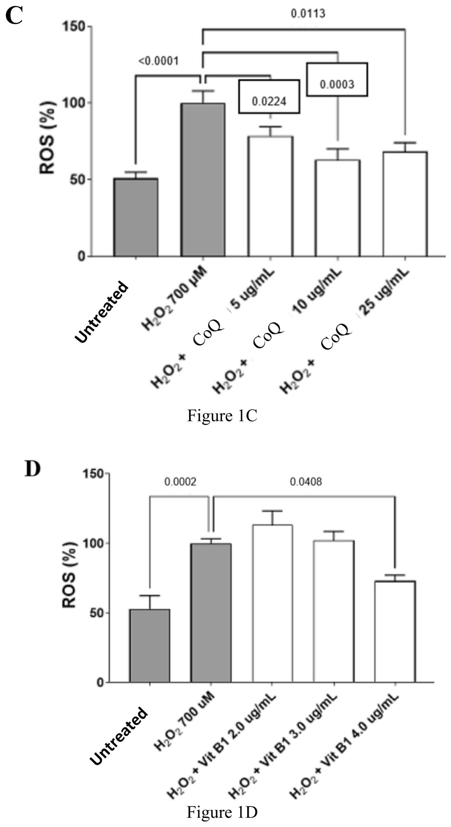

Figure 1C - Bar chart showing the percentage of ROS in the absence of treatment (first column from the left, untreated), after treatment with 700 µM H₂O₂ (second column from the left) and following the combination of 700 µM H₂O₂ with the starting material comprising coenzyme Q10 (indicated as "CoQ10") at 5, 10 and 25 µg/mL (white columns). The numbers indicated at the solid lines relate to the statistical data.
Figure 1D - Bar chart showing the percentage of ROS in the absence of treatment (first column from the left, untreated), after treatment with 700 µM H₂O₂ (second column from the left) and following the combination of 700 µM H₂O₂ with Vitamin B1 at 2, 3 and 4 µg/mL (white columns). The numbers indicated at the solid lines relate to the statistical data.

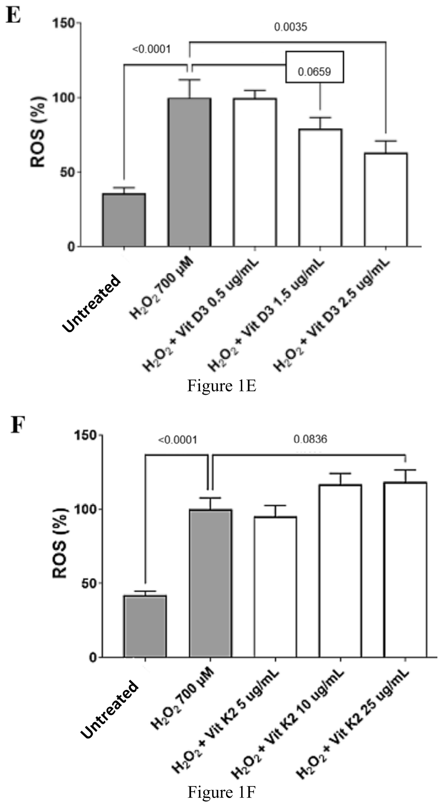

Figure 1E - Bar chart showing the percentage of ROS in the absence of treatment (first column from the left, untreated), after treatment with 700 µM H₂O₂ (second column from the left) and following the combination of 700 µM H₂O₂ with Vitamin D3 at 0.5, 1.5 and 2.5 µg/mL (white columns). The numbers indicated at the solid lines relate to the statistical data.
Figure 1F - Bar chart showing the percentage of ROS in the absence of treatment (first column from the left, untreated), after treatment with 700 µM H₂O₂ (second column from the left) and following the combination of 700 µM H₂O₂ with Vitamin K2 at 5, 10 and 25 µg/mL (white columns). The numbers indicated at the solid lines relate to the statistical data.

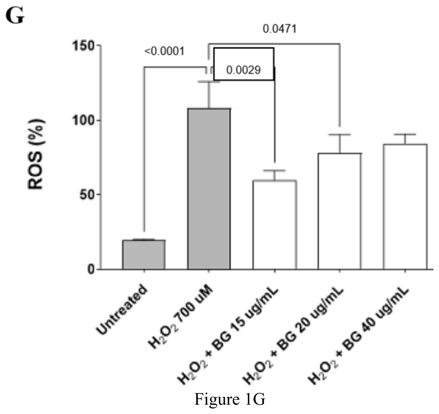

Figure 1G - Bar chart showing the percentage of ROS in the absence of treatment (first column from the left, untreated), after treatment with 700 µM H₂O₂ (second column from the left) and following the combination of 700 µM H₂O₂ with black garlic ("BG") at 15, 20 and 40 µg/mL (white columns). The numbers indicated at the solid lines relate to the statistical data.

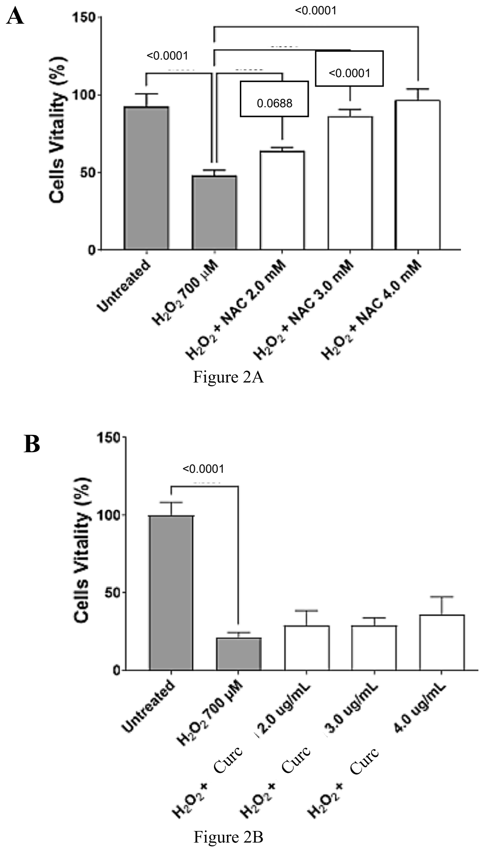

Figure 2A - Bar chart showing the percentage of vitality in the absence of treatment (first column from the left, untreated), after treatment with 700 µM H₂O₂ (second column from the left) and following the combination of 700 µM H₂O₂ with NAC at 2, 3 and 4 mM (white columns).
Figure 2B - Bar chart showing the percentage of vitality in the absence of treatment (first column from the left, untreated), after treatment with 700 µM H₂O₂ (second column from the left) and following the combination of 700 µM H₂O₂ with the starting material comprising Curcumin (indicated as "Cure") at 2, 3 and 4 µg/mL (white columns).

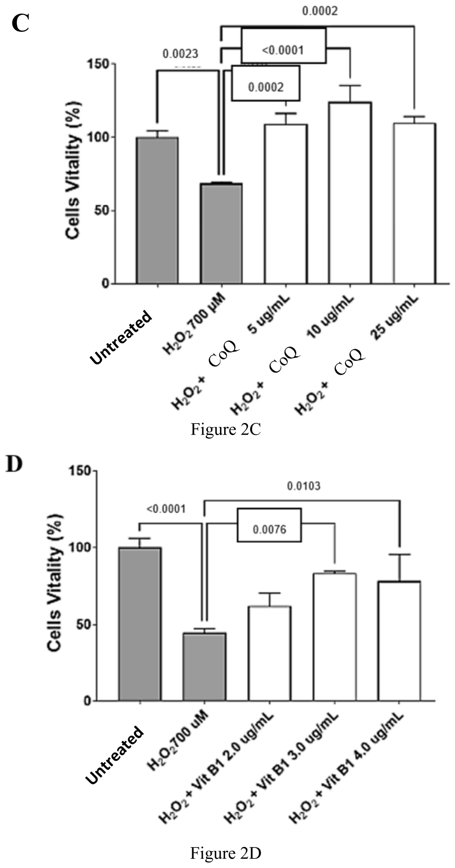

Figure 2C - Bar chart showing the percentage of vitality in the absence of treatment (first column from the left, untreated), after treatment with 700 µM H₂O₂ (second column from the left) and following the combination of 700 µM H₂O₂ with the starting material comprising coenzyme Q10 (indicated as "CoQ10") at 5, 10 and 25 µg/mL (white columns).
Figure 2D - Bar chart showing the percentage of vitality in the absence of treatment (first column from the left, untreated), after treatment with 700 µM H₂O₂ (second column from the left) and following the combination of 700 µM H₂O₂ with Vitamin B1 at 2, 3 and 4 µg/mL (white columns).

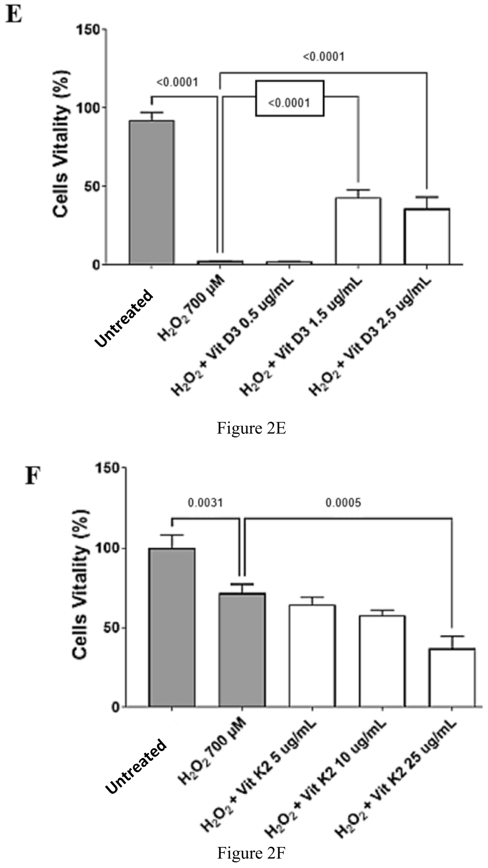

Figure 2E - Bar chart showing the percentage of vitality in the absence of treatment (first column from the left, untreated), after treatment with 700 µM H₂O₂ (second column from the left) and following the combination of 700 µM H₂O₂ with Vitamin D3 at 0.5, 1.5 and 2.5 µg/mL (white columns).
Figure 2F - Bar chart showing the percentage of vitality in the absence of treatment (first column from the left, untreated), after treatment with 700 µM H₂O₂ (second column from the left) and following the combination of 700 µM H₂O₂ with Vitamin K2 at 5, 10 and 25 µg/mL (white columns).

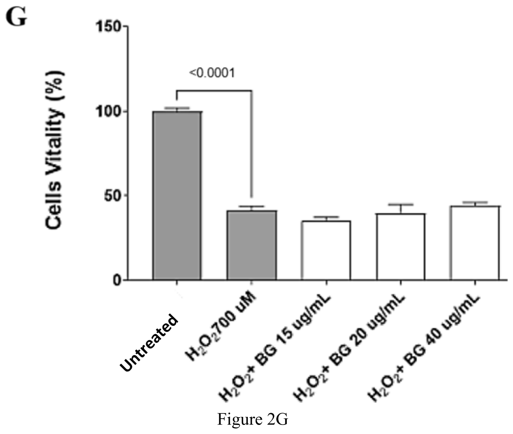

Figure 2G - Bar chart showing the percentage of vitality in the absence of treatment (first column from the left, untreated), after treatment with 700 µM H₂O₂ (second column from the left) and following the combination of 700 µM H₂O₂ with black garlic ("BG") at 15, 20 and 40 µg/mL (white columns).

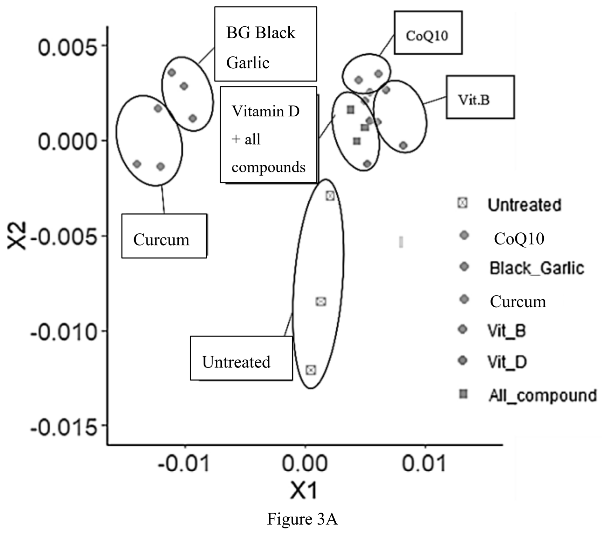

Figure 3A - Graph showing the multidimensional scaling of the data where each point represents a sample.

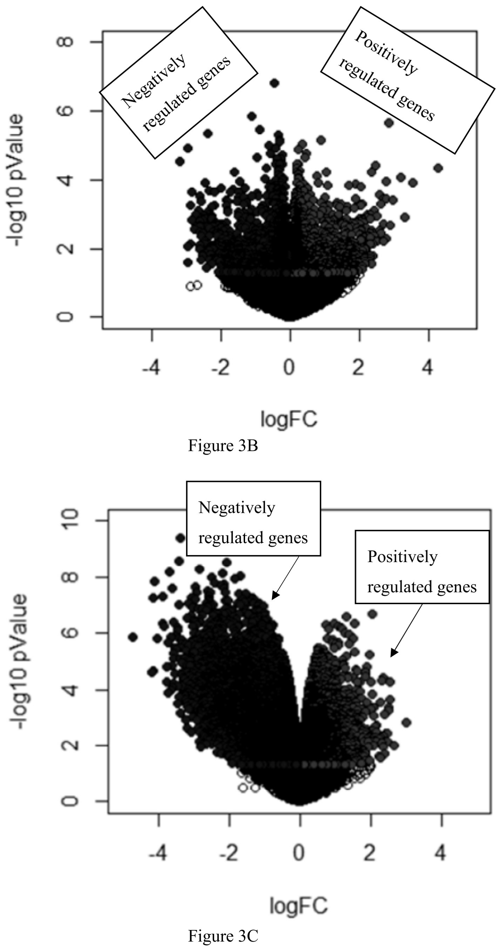

Figure 3B - Volcano plot representing the results obtained from the differential analysis between the samples treated with the combination of all compounds versus those in the basal, untreated condition.
Figure 3C - Volcano plot representing the results obtained from the differential analysis between the samples treated with the combination of all compounds versus the gene expression of the sum of the single compounds. The black dots (left) represent the down-regulated genes (adjusted p < 0.05 and logFC < 0), while the dark gray dots (right) represent the up-regulated genes (adjusted p < 0.05 and logFC > 0).
Figures 3D, 3E, 3F - Bar charts showing the expression, in terms of counts per million (CPM), of genes associated with anti-atherosclerosis, anti-oxidative stress and anti-inflammatory molecular pathways in 7 different conditions, basal (untreated), single compounds, and combination of all compounds (* adjusted p < 0.05, ** adjusted p < 0.01 and *** adjusted p < 0.001).

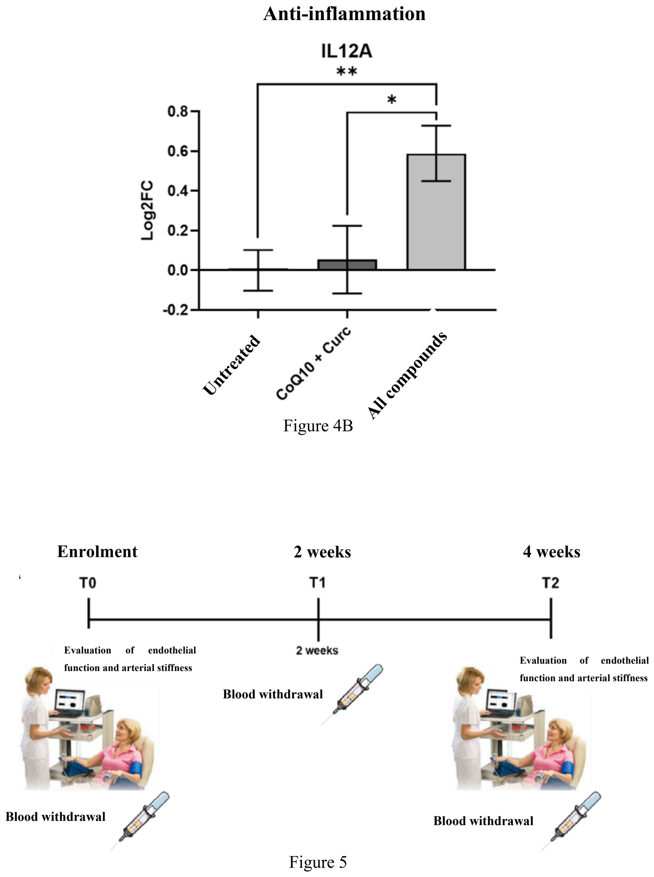

Figures 4A, 4B: Bar charts showing the expression, in terms of log2FC (fold-change), of genes associated with anti-oxidative stress (GADL1) and anti-inflammatory (IL12A) molecular pathways in the basal condition (untreated), in the double treatment with Coenzyme Q10 and Curcuma (CoQ10 + Curc) and in the combination of all the compounds that constitute the association of the invention ("All compounds"). The data were normalized on the gene expression of RPLP0 (housekeeping) (* p < 0.05 and ** p < 0.01).
Figure 5 - Diagram of the interventional clinical study with the supplement: T0 (Start): Enrollment of subjects, blood sampling to assess oxidative stress, evaluation of endothelial function and arterial stiffness, start of supplement intake. T1 (2 weeks): intermediate blood sampling to assess oxidative stress. T2 (4 weeks): final blood sampling and evaluation of endothelial function and arterial stiffness.

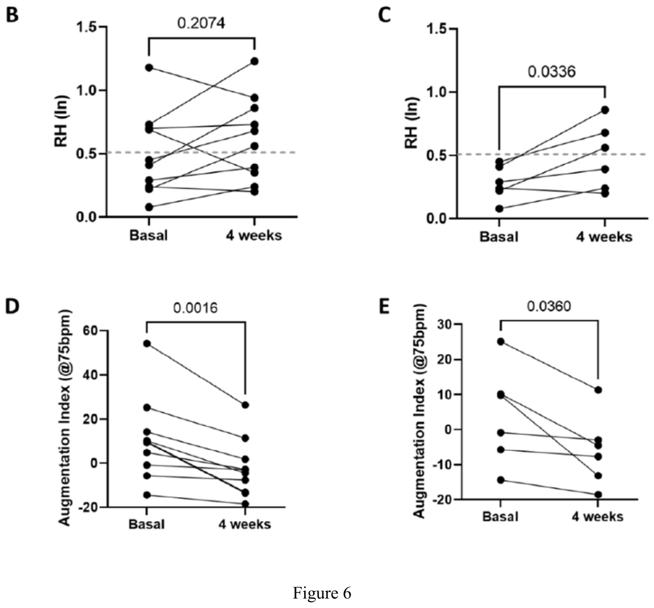

Figure 6 - Panel (B) is a line graph representing the reactive hyperemia index (endothelial functionality) measured by EndoPAT in the total population, comparing baseline values with those at 4 weeks. The dashed line represents the normality threshold of the RHI (Reactive Hyperemia Index), calculated as the ratio between the PAT^{®} signal measured after and before occlusion on the occluded side, normalized to the control side and corrected for baseline vascular tone (lnRHI > 0.51). Panel (C) is a line graph representing the Reactive Hyperemia Index (endothelial functionality) measured by EndoPAT in the population with endothelial dysfunction, comparing baseline values with those at 4 weeks. The dashed line represents the normality threshold of the RHI (Reactive Hyperemia Index), calculated as the ratio between the PAT^{®} signal measured after and before occlusion on the occluded side, normalized to the control side and corrected for baseline vascular tone (lnRHI > 0.51). Panel (D) is a line graph representing the arterial stiffness augmentation index measured by EndoPAT in the total population, comparing baseline values with those at 4 weeks. Panel (E) is a line graph representing the arterial stiffness augmentation index measured by EndoPAT in the population with endothelial dysfunction, comparing baseline values with those at 4 weeks.

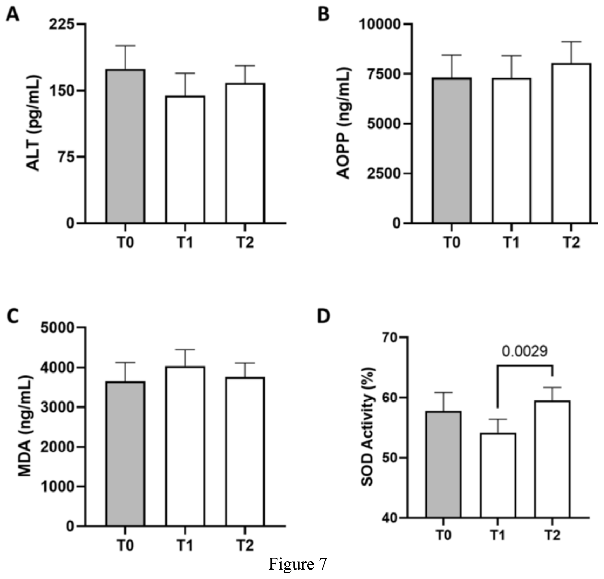

Figure 7 - Panel (A) is a bar chart representing the levels of alanine aminotransferase (ALT) at the time of enrollment (T0; gray bar), after 2 weeks (T1) and 4 weeks (T2) of supplement intake. Panel (B) is a bar chart representing the levels of advanced oxidation protein products (AOPP) at the time of enrollment (T0, gray bar), after 2 weeks (T1) and 4 weeks (T2) of supplement intake. Panel (C) is a bar chart representing the levels of malondialdehyde (MDA) at the time of enrollment (T0, gray bar), after 2 weeks (T1) and 4 weeks (T2) of supplement intake. Panel (D) is a bar chart representing the percentage of enzymatic activity of superoxide dismutase (SOD) at the time of enrollment (T0, gray bar), after 2 weeks (T1) and 4 weeks (T2) of supplement intake.

### DETAILED DESCRIPTION OF THE INVENTION

In the following, the invention and its preferred embodiments are described in more detail. It should be noted that, although the structure has been organized into paragraphs and subparagraphs, the information contained in each paragraph or subparagraph is not isolated, and may be combined with that contained in other paragraphs or, more generally, with other information contained in the text of the patent application.

For the purposes of the present invention, by extracts are meant, in general, products of natural origin that are obtained from plants and medicinal herbs of various types. The parts of the plant that can be used to obtain these products are multiple: leaves, flowers, fruits (pulp and/or peel), seeds, roots, rhizomes, bark, etc. They can be preparations in liquid form, solid form (dry extracts) or of intermediate consistency (soft extracts), obtained by means of suitable extraction processes; the latter include the use of appropriate solvents, supercritical extraction, the use of maceration or percolation, or other suitable procedures.

By dry extract is meant a solid preparation obtained through an extraction process using solvents that extract the active principles contained in the extract itself, and a subsequent solvent evaporation phase. Generally, the dry extract is characterized by a dry residue of not less than 90% by mass and has a moisture content generally less than or equal to 5% by mass.

### Association of active ingredients

The association of active ingredients defines a combination or mixture of active ingredients useful for the medical purposes of the invention.

The association of active ingredients comprises or consists of
- dry extract of Curcuma,
- dry extract of fermented black garlic,
- coenzyme Q10,
- at least one vitamin selected from the group consisting of: at least one B-group vitamin; vitamin D; and combinations of the foregoing.

### Dry extract of Curcuma

Preferably, the dry extract of Curcuma is a dry extract of *Curcuma longa.*

Preferably, the dry extract of Curcuma is obtained from the naturally dried rhizome of *Curcuma longa.*

Preferably, the dry extract of Curcuma contains curcuminoids. Preferably, the curcuminoids are in an amount comprised between 10% and 50% by weight, preferably between 10% and 40% by weight, preferably between 10% and 30% by weight, preferably of 20% by weight, based on the total weight of the dry extract of Curcuma.

Preferably, the dry extract of Curcuma is in an amount comprised between 5% and 17% by weight, preferably between 8% and 15% by weight, preferably between 10% and 15% by weight, preferably between 10% and 13% by weight, preferably between 11% and 13% by weight, based on the total weight of the association.

### Dry extract of fermented black garlic

Preferably, the dry extract of black garlic is obtained from the bulb of *Allium sativum L.*

The dry extract of black garlic is obtained from the fermentation of the bulbs; these are left to ferment in an environment with controlled humidity and temperature - a temperature comprised between 60°C and 90°C and with a humidity rate comprised between 80 and 90% - for one month. After 30 days, another six weeks of oxidation will follow, which will lead the garlic to blacken and soften. Once softened, it is subjected to grinding, then to extraction in the presence of water. In this sense, the dry extract of black garlic represents a fermented dry extract.

Preferably, the dry extract of black garlic contains S-allyl cysteine. Preferably, S-allyl cysteine is in an amount comprised between 0.05% and 0.5% by weight, preferably between 0.07% and 0.3% by weight, preferably between 0.09% and 0.15% by weight, preferably of 0.1% by weight, based on the total weight of the dry extract of black garlic.

Preferably, the dry extract of black garlic is in an amount comprised between 30% and 50% by weight, preferably between 35% and 45% by weight, preferably between 37% and 43% by weight, preferably between 40% and 43% by weight, based on the total weight of the association.

### Coenzyme Q10

Preferably, the coenzyme Q10 is present in a starting material that contains it in combination with suitable excipients. Preferably, the excipients are selected from the group consisting of: chemical derivatives of cellulose, for example hydroxypropylcellulose; sucrose esters; and combinations of the foregoing. Preferably, the coenzyme Q10 is present in said starting material in an amount comprised between 10% and 35% by weight, preferably between 15% and 30% by weight, preferably between 20% and 30% by weight, preferably equal to 25% by weight, based on the total weight of the starting material.

Preferably, the coenzyme Q10, preferably in the form of said starting material, is present in an amount comprised between 20% and 35% by weight, preferably between 25% and 33% by weight, preferably between 27% and 30% by weight, preferably between 28% and 29% by weight, by weight based on the total weight of the association.

### At least one vitamin

The at least one vitamin is selected from the group consisting of: at least one B-group vitamin; vitamin D; and combinations of the foregoing.

Preferably, the at least one B-group vitamin is intended as a vitamin selected from the group consisting of: thiamine (or vitamin B1); riboflavin (or vitamin B2); niacin (or vitamin B3); pantothenic acid (or vitamin B5); vitamin B6; biotin (or vitamin B8); folic acid (or vitamin B9); cobalamin (or vitamin B12); and combinations of the foregoing.

Preferably, the at least one B-group vitamin is thiamine.

Preferably, the at least one vitamin is in a total amount comprised between 6% and 25% by weight, preferably between 7% and 25% by weight, preferably between 7% and 20% by weight, preferably between 7% and 19% by weight, based on the total weight of the association.

Preferably, the at least one B-group vitamin is in an amount comprised between 6% and 20% by weight, preferably between 9% and 20% by weight, preferably between 10% and 19% by weight, preferably between 11% and 19% by weight, based on the total weight of the association.

Preferably, the vitamin D is comprised in a starting material. Preferably, the starting material can also contain excipients, for example selected from the group consisting of: carriers, such as modified starch and/or sucrose and/or vegetable oils; antioxidants; flow agents.

Preferably, the vitamin D is present in the form of cholecalciferol or vitamin D3.

Preferably, the starting material comprising the vitamin D is in an amount between 2% and 13% by weight, preferably between 4% and 13% by weight, preferably between 5% and 10% by weight, preferably between 5% and 8% by weight, preferably between 6% and 8% by weight, based on the total weight of the association.

Preferably, the cholecalciferol is in an amount comprised between 0.1% and 0.4% by weight, preferably between 0.1% and 0.3% by weight, preferably between 0.2% and 0.3% by weight, based on the total weight of the starting material comprising said vitamin D.

Preferably, the at least one vitamin is a combination of thiamine and vitamin D in the form of cholecalciferol.

### Weight ratios

Preferably, the weight ratio between the amount of coenzyme Q10 and the amount of the dry extract of Curcuma is comprised between 1:1.5 and 1:5, preferably between 1:2 and 1:4, preferably between 1:2 and 1:3, preferably of 1:2.5.

Preferably, the weight ratio between the amount of the dry extract of black garlic and the amount of the dry extract of Curcuma is comprised between 1:2 and 1:7, preferably between 1:2 and 1:5, preferably between 1:3 and 1:4, preferably of 1:3.75.

Preferably, the weight ratio between the amount of the dry extract of black garlic and the amount of coenzyme Q10 is comprised between 1:1.2 and 1:4, preferably between 1:1.2 and 1:3, preferably between 1:1.2 and 1:2, preferably of 1:1.5.

Preferably, the weight ratio between the sum of the amounts of dry extract of Curcuma, coenzyme Q10 and dry extract of black garlic and the amount of the at least one vitamin is comprised between 1:2 and 1:7, preferably between 1:2 and 1:5, preferably between 1:3 and 1:5, preferably of 1:4.4.

It is noted that such weight ratios contribute to the effect of treating the pathological condition of dysfunction of the vascular and/or valvular endothelium and/or of maintaining the homeostasis of the vascular and/or valvular endothelium.

### Medical use of the association of active ingredients

The association is for use in restoring and/or maintaining the physiological function of the vascular and/or valvular endothelium of the cardiocirculatory system.

By "restoring the physiological function of the vascular and/or valvular endothelium of the cardiocirculatory system" is meant the ability to restore the physiological function in altered or pathological conditions of the vascular and/or valvular endothelium of the cardiocirculatory system.

By "maintaining the function of the vascular and/or valvular endothelium of the cardiocirculatory system" is meant the ability to preserve the structural integrity and physiological functioning of the vascular and/or valvular endothelium. The maintenance of the function of the vascular and/or valvular endothelium of the cardiocirculatory system may be required, for example, in case of inflammation of the endothelium; an increase in the rigidity of the vascular and/or valvular tone with respect to the physiological condition; damage to the endothelium; an increase in reactive oxygen species with respect to the physiological condition; vascular and/or valvular calcification; and mixtures of the foregoing.

Preferably, the restoration of the physiological function of the vascular and/or valvular endothelium is subordinate to a dysfunction of the vascular and/or valvular endothelium.

Preferably, a dysfunction of the vascular and/or valvular endothelium manifests concomitantly with or is caused by a pathological state selected from the group consisting of: inflammation of the endothelium; an increase in the rigidity of the vascular and/or valvular tone with respect to the physiological condition; damage to the endothelium; an increase in reactive oxygen species with respect to the physiological condition; vascular and/or valvular calcification; and mixtures of the foregoing.

Preferably, the restoration of the physiological function of the vascular and/or valvular endothelium is subordinate to a dysfunction of the vascular and/or valvular endothelium that manifests concomitantly with cardiovascular pathologies selected from the group consisting of: aortic valve sclerosis; aortic stenosis; coronary artery diseases; and mixtures of the foregoing.

Preferably, the association is for use in the treatment of a pathological condition of dysfunction of the vascular and/or valvular endothelium of the cardiocirculatory system and/or in the maintenance of the function of the vascular and/or valvular endothelium of the cardiocirculatory system.

By pathological condition of dysfunction of the vascular and/or valvular endothelium is meant a pathological condition that can manifest concomitantly with (or be caused by) a dysfunction of the vascular and/or valvular endothelium.

Preferably, the dysfunction of the vascular and/or valvular endothelium manifests concomitantly with cardiovascular pathologies.

Preferably, the cardiovascular pathologies are selected from the group consisting of: aortic valve sclerosis; aortic stenosis; coronary artery diseases; and mixtures of the foregoing.

Preferably, the association is for use in the treatment and/or prevention of the dysfunction of the vascular and/or valvular endothelium of the cardiocirculatory system, for example that is caused by or manifests concomitantly with a condition selected from the group consisting of: an inflammatory condition of the vascular and/or valvular endothelium; cardiovascular pathologies; and combinations of the foregoing.

### Oral formulation comprising the association of active ingredients

Preferably, the oral formulation comprises
- the association for use described in the preceding sections,
- suitable excipients and/or diluents.

Preferably, the association is the only combination or mixture of active ingredients comprised in the formulation.

Preferably, the association is in an amount comprised between 20% and 60% by weight, preferably between 30% and 60% by weight, preferably between 30% and 50% by weight, preferably between 35% and 50% by weight, preferably between 35% and 45% by weight, preferably between 40% and 45% by weight, based on the total weight of the formulation.

Preferably, the dry extract of Curcuma is in an amount comprised between 2% and 7% by weight, preferably between 3% and 7% by weight, preferably between 3% and 5% by weight, preferably between 4% and 5% by weight, preferably equal to 4.8% by weight, based on the total weight of the formulation.

Preferably, the dry extract of black garlic is in an amount comprised between 13% and 25% by weight, preferably between 13% and 20% by weight, preferably between 15% and 20% by weight, preferably between 17% and 19% by weight, preferably equal to 18% by weight, based on the total weight of the formulation.

Preferably, the coenzyme Q10 is in an amount comprised between 8% and 17% by weight, preferably between 8% and 15% by weight, preferably between 10% and 13% by weight, preferably between 11% and 13% by weight, preferably equal to 12% by weight, based on the total weight of the formulation.

Preferably, the at least one vitamin is in an amount comprised between 2% and 12% by weight, preferably between 2% and 10% by weight, preferably between 2% and 8% by weight, preferably between 3% and 8% by weight, preferably equal to 7.8% by weight, based on the total weight of the formulation.

Preferably, the at least one B-group vitamin, for example thiamine, is in an amount comprised between 2% and 7% by weight, preferably between 3% and 7% by weight, preferably between 3% and 5% by weight, preferably between 4% and 5% by weight, preferably equal to 4.8% by weight, based on the total weight of the formulation. Preferably, the vitamin D is in an amount comprised between 1% and 7% by weight, preferably between 1% and 5% by weight, preferably between 2% and 5% by weight, preferably between 2% and 4% by weight, preferably equal to 3% by weight, based on the total weight of the formulation.

Preferably, the formulation is in a solid form.

Preferably, the formulation is in a solid form selected from the group consisting of: powder, granulate, tablet, capsule, sachet or single-dose pouch; preferably, the formulation is a tablet.

Preferably, the suitable excipients and/or diluents are those known in the state of the art and suitable for the preparation of formulations, preferably of solid formulations. Preferably, the suitable excipients and/or diluents are selected from the group consisting of: bulking agents, thickeners, glidants, exfoliating agents, lubricants, flow agents, disintegrants, flavoring agents, pH adjusters, sweeteners, gelatin, plasticizers, solvents, silicon derivatives, and combinations of the foregoing.

Preferably, the suitable excipients and/or diluents are selected from the group consisting of: microcrystalline cellulose; dicalcium phosphate dihydrate; croscarmellose sodium; silicon dioxide; magnesium stearate; and mixtures of the foregoing.

Preferably, the formulation may comprise at least one coating layer that partially or completely surrounds the formulation. In this sense, the formulation is referred to as a coated formulation.

Preferably, the coating is gastro-resistant.

Preferably, the coating layer comprises coating excipients known to a person skilled in the art, for example they are selected from the group consisting of: excipients that contribute to gastro-resistance; opacifiers; colorants; plasticizers; film-forming agents; excipients that contribute to taste masking; waterproofing agents; sweeteners; sugars and polyols; gums; cellulose derivatives; polymers; fatty acids.

Preferably, the coating excipients are selected from the group consisting of: Ethylcellulose (E462); HPMC (E464); Talc (E553b); Calcium carbonate (E170); Sodium alginate (E401); Medium-chain triglycerides; PEG (E1521); Stearic acid (E570); Yellow iron oxide (E172(iii)); and mixtures of the foregoing.

Preferably, the formulation can be taken 1 or 2 times a day, preferably 1 time a day. Preferably, the formulation is for use as a food supplement, nutraceutical, medicinal specialty for human or animal use.

By food supplement is meant a formulation that falls within the definition provided by Directive 2002/46/EC and subsequent amendments. In this regulation, food supplements are defined precisely as: "foodstuffs the purpose of which is to supplement the normal diet and which are concentrated sources of nutrients or other substances with a nutritional or physiological effect, alone or in combination, marketed in dose form, namely forms such as capsules, pastilles, tablets, pills and other similar forms, sachets of powder, ampoules of liquids, drop dispensing bottles, and other similar forms of liquids and powders designed to be taken in measured small unit quantities".

The use of extracts and plant preparations in food supplements is currently regulated in Italy by the Decree of the Ministry of Health of 10 August 2018. Annex 1 of said DM contains the list of permitted plants and their parts, accompanied where appropriate by supplementary provisions for use. This list is applicable only to plants/parts of plants and their derivatives (e.g., extracts or other preparations) with a history of significant food consumption before 1997. With Directorial Decree no. 12 of 13/12/2023, Annex 1 was subject to a new amendment intended to specify the meaning of the indication "aetheroleum," contained in the column "traditionally used part", in order to correctly identify the usable part in food supplements by correlating it to the significant use as defined by Regulation 2015/2283. EC Regulation 1170/2009 instead governs the vitamins and minerals permitted in food supplements.

By nutraceutical is meant one or more components or active principles of foods that have positive effects on health, prevention, and treatment of diseases.

By medicinal specialty is meant a pre-packaged pharmaceutical form, produced industrially and authorized on the basis of documentation containing the experimental chemical, biological, pharmaceutical, pharmaco-toxicological, and clinical results relating to the drug that is placed on the market with a special name (registered trademark).

### EXAMPLES

Hereinafter, the Applicant provides examples for illustrative but not limiting purposes.

### Example 1 - Formulation in the form of a food supplement comprising the association of active ingredients of the invention

The formulation is in tablet form.

| **Composition** | **mg per tablet** | **% by weight on the total weight of the tablet (% w/w)** | **% NRV*** |
|---|---|---|---|
| Curcuma dry extract 20% curcuminoids | 24 | 4.80 | / |
| Starting material containing coenzyme Q10 at 25% by weight on the total weight of the starting material, in combination with hydroxypropylcellulose and sucrose esters | 60 | 12.0 | / |
| Fermented black garlic dry extract 0.1% S-allyl cysteine | 90 | 18.0 | / |
| Thiamine HCl (vit.B1) | 24 | 4.80 | 1716 |
| Vitamin D3 100,000 IU/g powder | 15 | 3.00 | 750 |
| Microcrystalline cellulose | 135 | 27.0 | / |
| Dicalcium phosphate dihydrate | 115 | 23.0 | / |
| Croscarmellose sodium | 5.00 | 1.00 | / |
| Silicon dioxide | 24.0 | 4.80 | / |
| Magnesium stearate | 8.00 | 1.60 | / |
| **Total** | **500** | **100** | |
| **Nutrient Reference Values* | | | |

It is noted that the formulation in tablet form may or may not be coated. When coated, the coating may have the following composition:

| **Coating component** | **mg per tablet** |
|---|---|
| Ethylcellulose (E462) | 9.94 |
| HPMC (E464) | 9.74 |
| Talc (E553b) | 3.94 |
| Calcium carbonate (E170) | 3.94 |
| Sodium alginate (E401) | 2.31 |
| Medium-chain triglycerides | 2.11 |
| PEG (E1521) | 2.07 |
| Stearic acid (E570) | 1.19 |
| Yellow iron oxide (E172(iii)) | 1.04 |

### Example 2 - Efficacy evaluation test of the association of active ingredients.

### 2.1 Materials and methods

### 2.1.1. Active ingredients for in vitro tests

N-acetylcysteine - Powder
Starting material comprising Curcumin - Powder tit. 20% curcuminoids
Vitamin D3 - Powder 100,000 IU/g
Starting material comprising coenzyme Q10 - Powder tit. 23.8 - 26.3% Ubiquinol
Vitamin K2 - Powder tit. 0.2% menaquinone
Vitamin B1 HCl - Powder
Black Garlic (ALLICYS) - Powder 0.1% S-Allyl-L-cysteine

### 2.1.2. Isolation of valvular endothelial cells

The valvular endothelial cells (VECs) were isolated from pathological human aortic valve leaflets explanted following valve replacement surgery. After washing with approximately 5 mL of sterile phosphate buffer (PBS-BioWhittaker Lonza, Belgium), the leaflets were placed in a tube containing 3 mL of Advanced Dulbecco's modified Eagle (Advanced DMEM, Life Technologies, Italy), containing 1'1% penicillin/streptomycin, 1'1% L-glutamine and 10% fetal bovine serum (FBS Fetal Bovine Serum, Gibco, Thermo Fisher, Italy). Subsequently, 300 µL of type II collagenase (Worthington Biochemical Corp.) were added to promote the detachment of endothelial cells. The tube was then placed for 20 minutes at 37°C in a humidified incubator with a saturated atmosphere of 5% CO₂. Following this incubation, using a pipette to achieve complete detachment of the endothelial cells, the liquid containing medium and collagenase was directed onto both sides of the valve leaflets. At the end of this operation, the liquid containing the endothelial cell suspension was recovered in a tube and centrifuged at 432 x g for 5 minutes at room temperature. The pellet thus obtained was then resuspended in 1 mL of Medium 200 (Gibco, Thermo Fisher, Italy) containing 1'1% penicillin/streptomycin, 1'1% L-Glutamine, growth factors (Low Serum Growth Supplement - LSGS; Thermo Fisher, Italy) and 10% FBS. At this point, in order to select exclusively endothelial cells, magnetic beads conjugated with CD31 (Dynabeads CD31 Endothelial Cell, Invitrogen, Italy), an endothelial marker, were used. In particular, the cell suspension was transferred to an eppendorf tube containing 15 µl of previously washed magnetic beads, and placed on a rotator for 20 minutes at 4°C to allow the formation of the bond between the beads and the cells. At the end of the incubation, using a magnet, 3 washes with PBS were performed and after resuspending the pellet in 1 mL of M200, the endothelial cells thus obtained were cultured in 35 mm Petri dishes pre-treated with a sterile aqueous solution of 0.1% gelatin (Sigma-Aldrich) to be then expanded in larger Petri dishes for carrying out all the necessary experiments.

### 2.1.3. Detection of reactive oxygen species and intracellular nitric oxide

The VECs were seeded at a density of 4000 cells/well in a 96-well plate, the surface of which was previously coated with 0.1% gelatin. To ensure that all plated cells reached the same phase of the cell cycle, the VECs were subjected to one night of "starvation" with FBS-free medium. The next day, the medium was replaced with complete M200 and the cells were subjected to the following treatments: no treatment (negative control), hydrogen peroxide (H₂O₂) 700 µM (positive control for oxidative stress), H₂O₂ 700 µM with the addition of N-acetyl cysteine (2, 3 and 4 mM), starting material comprising Curcumin (2, 3 and 4 µM), starting material comprising coenzyme Q10 (5, 10, 25 µg/mL), Vitamin B1 (2, 3 and 4 µg/mL), Vitamin D3 (0.5, 1.5 and 2.5 µg/mL), Vitamin K (5, 10 and 25 µg/mL) and black garlic (15, 20 and 40 µg/mL), added individually or in combination. The treatment lasted 1 hour, after which the medium was replaced with complete medium and the cells were subjected to a 48-hour "recovery" period. The detection of reactive oxygen species (ROS) was performed with the CellROX^{®} green Assay Kit (Thermo Fisher Scientific) following the instructions provided by the manufacturer. Differently, the production of nitric oxide (NO) was evaluated using the compound 4-Amino-5-Methylamino-2',7'-Difluorofluorescein Diacetate (DAF-FM, Thermo Fisher Scientific). In both cases, the analysis was performed using an automated live-cell imaging system (IncuCyte S3, Sartorius).

### 2.1.4. Cell viability assay

Following the evaluation of ROS production described in the previous section, the thus treated cells were subjected to the quantitative colorimetric assay which involves the use of 3-(4,5-dimethylthiazol-2-yl)-2,5-diphenyltetrazolium bromide (MTT) to evaluate cell viability following the treatments. For this evaluation, the cells were then treated for 4 hours with a solution of MTT (5mg/ml) diluted 1:10 in Ad DMEM (0.5% FBS).

After incubation, the MTT solution, formed by a tetrazolium salt that is transformed into formazan crystals only by viable cells, was removed and the crystals were solubilized using dimethyl sulfoxide (DMSO, Sigma-Aldrich), which allows the formation of a purple solution. The intensity of this coloration is directly proportional to cell viability and was evaluated by spectrophotometric reading of the absorbance at two different wavelengths, 590 and 620 nm (Infinite M200 Pro, Tecan Trading AG).

### 2.1.5. Poly-adenylated RNA sequencing

The VECs were seeded at a density of 150,000 cells per 60 mm dish previously gelatinized with 0.1% gelatin. Once sub-confluence was reached, the cells were treated in the same manner and time frames described in the previous sections, at the concentrations identified as best for reducing oxidative stress and maintaining good viability. At the end of the treatment, the cells were lysed with 350 µL of RL buffer (NORGEN BIOTEK CORP., Canada) using a "cell scraper". The cell lysate thus obtained was then transferred to RNase-free tubes to proceed with RNA extraction using the commercial Total RNA Purification Plus Kit (NORGEN BIOTEK CORP., Canada) following the instructions provided by the manufacturer. Briefly, first, genomic DNA was removed using specific columns (gDNA Removal Columns) and centrifugation at 14000 x g for 1 minute. To the eluate thus obtained, 210 µL of 100% ethanol were then added. Subsequently, the RNA, made insoluble by the addition of ethanol, was isolated by binding to the resin contained in further columns provided by the kit (RNA Purification Columns), after centrifugation at 3500 x g for 1 minute. Subsequently, the resin was subjected to three washes with 400 µL of Wash Solution A, centrifuging at 14000 x g for 1 minute after each wash. At the end of this phase, the RNA was then eluted in 32 µL of elution solution supplied with the kit, quantified using the Infinite M200 Pro plate reader (Tecan Trading AG) and subsequently stored at -20°C. Before proceeding with the sequencing process, 10 µg of total RNA were treated with DNase using the TURBO DNA-free^{™} kit (ThermoFisher Scientific). The RNA obtained was then precipitated in pure ethanol and ammonium acetate (125 mM), washed with 70% ethanol and resuspended in deionized water, to ensure its purity, before being quantified by the microplate reader mentioned above.

At this point, the libraries were prepared and sequenced by Eurofins Genomics (Ebersberg, Germany) using Illumina technology. The files containing the sequenced reads (fastq paired-end) were pre-processed in a Linux/Unix environment. In particular, for each sample, the alignment of the reads to the human reference genome (Ensembl GRCh38) was performed using the STAR software (v2.7.10b) [19] and only those uniquely expressed were counted using FeatureCounts (v2.0.6) [20]. The raw count matrix was then imported and analyzed in R. Using the DaMiRseq package [21], genes with low expression (a number of associated reads less than 3 in at least 30% of the samples) were filtered, the counts per million (CPM) were calculated in logarithm, and finally the values were adjusted for the identified surrogate variables, in order to eliminate the presence of technical effects.

### 2.1.6. Clinical study

The tablets of the supplement, the best mixture of compounds identified through the *in vitro* studies, were administered to 10 volunteers.

These volunteers met the following inclusion criteria: healthy subjects, without known pathologies, aged between 20 and 65 years, of both sexes, and with signed informed consent.

Subjects under 20 or over 65 years of age, with confirmed cardiovascular, oncological, immunological, or neurodegenerative pathologies, or who were taking antioxidant food supplements, were not enrolled, as these characteristics were considered as exclusion criteria.

All participants took the supplement once a day at the same time for 28 days (4 weeks). Before intake (baseline), at 2 weeks from intake, and at 4 weeks, blood samples were taken to evaluate a series of circulating oxidative stress markers.

At baseline and at a distance of 4 weeks, the evaluation of endothelial function and arterial thickness were evaluated by calculating the reactive hyperemia index (RHI) and the arterial stiffness augmentation index using EndoPAT^{®} technology.

### 2.1.7. Enzyme-linked immunosorbent assays (ELISA)

Peripheral venous blood samples were collected from the enrolled subjects at the times described in the previous section. For the collection, tubes containing EDTA (9.3 mM; Vacutainer Systems, Becton Dickinson, Franklin Lakes, NJ, USA) were used, which were immediately placed on ice and centrifuged at 3,000 x g at 4 C° within 30 minutes of collection. In this way, the plasma was separated and the aliquots were stored at - 80°C until the execution of the assays.

On these samples, ELISA assays were performed to evaluate the detection of variations in: alanine aminotransferase (ALT; Human ALT SimpleStep ELISA^{®} Kit; Abcam), advanced oxidation protein products (AOPP; Human Advanced Oxidation Protein Products ELISA; Abbexa), and malondialdehyde (MDA; Malondialdehyde ELISA Kit; Abcam).

Furthermore, a colorimetric assay was also performed for the evaluation of the enzymatic activity of superoxide dismutase (SOD; Superoxide Dismutase Activity Assay Kit; Abcam).

All assays were performed following the instructions provided by the manufacturers.

### 2.1.8. Statistical analysis

The statistical analysis was conducted using GraphPad Prism software (version 9). The data were reported as mean + standard error and the comparisons between groups were based on the t-test or paired t-test for baseline and follow-up comparisons.

When more than two groups were compared, the ANOVA test was used as an omnibus statistic followed by Fisher's LSD test.

In all analyses, two-tailed p-values lower than 0.05 were considered statistically significant. The analysis of differentially expressed genes was conducted using the limma package in R. Genes with an adjusted p < 0.05 and logFC<>0 were defined as up-regulated and down-regulated, respectively. The t-test was performed to analyze the difference in gene expression across the various conditions.

### 2.2. Study of the efficacy of the association of the invention in reducing oxidative stress and cell death of the valvular endothelium

Oxidative stress at the endothelial level is a crucial step in the development of the AVSc condition and its associated complications. While the antioxidant effect of N-acetylcysteine (NAC) on VECs is well documented [6, 13], and here confirmed at different concentrations both in terms of production of reactive oxygen species (ROS; Figure 1A) and of vitality (Figure 2A), for other potentially antioxidant compounds the effects on the ability to reduce this stress on the valvular endothelium are not known. Among the compounds whose antioxidant effects are not known, are included: the starting material comprising Curcumin, the starting material comprising coenzyme Q10, the extract of black garlic and vitamins B12, D3 and K.

To this end, the levels of ROS produced by the endothelium following treatment of VECs with hydrogen peroxide (H₂O₂), administered individually and in conjunction with the above-listed compounds at different concentrations, were measured.

The aim was to identify the best concentration capable of reducing the ROS level. The best concentrations identified for each compound are: 4 µg/mL for the starting material comprising Curcumin (Figure 1B), 10 µg/mL for the starting material comprising coenzyme Q10 (Figure 1C), 4 µg/mL for Vitamin B1 (Figure 1D), 2.5 µg/Ml for Vitamin D3 (Figure 1E) and 15 µg/mL for black garlic (Figure 1G).

No decrease in ROS production was observed following treatment with Vitamin K, which at the highest concentration used (25 µg/mL) conversely significantly increased their production (Figure 1F).

In parallel with the measurement of ROS, cell viability was also evaluated following the same treatments and it was observed that cell viability, at the concentrations listed above, was significantly increased or showed an upward trend when compared to treatment with H₂O₂ alone (Figures 2B-2E and 2G), showing how not only the use of the compound was able to reduce the oxidative stress caused by H₂O₂, but at the same time was able to improve cell viability by reducing its mortality rate.

In the case of Vitamin K, the increase in ROS at a concentration of 25 µg/mL was accompanied by a significant decrease in viability (Figure 2F), further supporting the concept that the presence of ROS and cell viability are strictly correlated.

### 2.3. Study of the synergistic efficacy of the association of the invention in enhancing a beneficial response for the valvular endothelium

A study was conducted on the effects produced by the five active compounds, both individually and in combination, through the evaluation of cellular gene transcripts.

The results obtained showed that the combination of the compounds significantly modulated thousands of genes both with respect to the control (i.e., with respect to the untreated sample, therefore in basal conditions), and with respect to the sum of the effects of the single compounds (Figures 3B and 3C).

In particular, Figure 3C shows that the simultaneous addition of all compounds positively regulated several genes with respect to the sum of the effects of the single compounds. This suggests a synergistic interaction between the compounds when combined, which is not observable when the compounds are applied individually. Furthermore, the simultaneous addition of all compounds regulated many genes negatively with respect to the sum of the effects of the single compounds. This suggests an antagonistic interaction between the compounds when combined, which is not observable when the compounds are applied individually.

In particular, the analysis brought to light significant and synergistic differences of the combination of the 5 active compounds with respect to the sum of the single active ingredients for three genes known to have anti-atherosclerotic (Gastric Inhibitory Polypeptide Receptor; GIPR) [14, 15], anti-oxidative stress (Glutamate Decarboxylase Like 1; GADL1) [16] and anti-inflammatory (Interleukin 12A; IL12A) [17, 18] effects (Figures 3D-F).

It should be noted that some of the active ingredients, such as, for example, the dry extract of Curcuma, when taken individually, show an effect on GIPR, GADL1 and IL12A that is even opposite to that of the association of active ingredients. This means that, individually, the compound has a gene-suppressing effect, which could be potentially negative for cellular homeostasis. However, in combination with the other compounds, the effect is to enhance the expression of these genes, resulting in a beneficial effect on valvular endothelial cells.

These results suggest that the combined use of these active compounds offers an approach to improve endothelial function and cardiovascular health in general.

Thus, the results of the test just discussed have shown that the combined use of the five compounds (black garlic, curcuma, coenzyme Q10, vitamin B1 and vitamin D3) determines an effect superior to the sum of the effects of the single components on the activation of specific genes with anti-oxidative stress (GADL1) and anti-inflammatory (IL12A) function. Therefore, the Applicant believes to have demonstrated the synergistic effect among all the compounds comprised in the association of the invention.

The Applicant has also verified that the effect of the mixture of the five compounds was superior to that of the combination/pair "coenzyme Q10-dry extract of Curcuma" through a real-time quantitative PCR analysis.

The analysis confirmed that the combination of the five compounds induces a significant increase in the expression of specific genes involved in protective processes for endothelial function and homeostasis. Furthermore, the gene activation of GADL1 (anti-oxidative stress) and IL12A (anti-inflammatory) was significantly higher following treatment with the five compounds compared to the pair of coenzyme Q10 and dry extract of Curcuma (Figures 4A and 4B).

Overall, these data demonstrate the superiority and the marked synergistic effect of the combination of the five selected compounds compared to the coenzyme Q10-curcuma pair.

### 2.4. Calculation of the hypothetically effective dosage of the active compounds

To find dosages that could be effective in an oral form through the intake of a tablet, the starting point was the relative concentrations from the final results of the *in vitro* study reported above.

The objective was to hypothetically arrive at a plasma concentration of each active ingredient as close as possible to that used in the *in vitro* study (which was in the order of micrograms per milliliter).

The quantities of each active principle were then calculated, considering that the volume of blood present in the human body is about 5 liters. The calculation was purely theoretical as it did not take into account several aspects, including: i) the kinetics of release of the active ingredients from the oral form (tablet) and ii) the *in vivo* bioavailability of the active ingredients.

In any case, the tablet was designed with a gastro-resistant coating to protect the active ingredients from possible degradation due to the digestive process that occurs at the gastric level. The release of the active ingredients therefore occurs in the intestinal environment, where the disintegration of the tablet is guaranteed in less than an hour by a suitable choice and combination of food additives that act as excipients. In particular, the excipients used were: microcrystalline cellulose, dicalcium phosphate dihydrate, precipitated silicon dioxide, amorphous silicon dioxide, magnesium stearate, croscarmellose sodium with a coating of hydroxypropylmethylcellulose, talc, calcium carbonate, polyethylene glycol, shellac and yellow iron oxide. Furthermore, the obtained product meets the Pharmacopoeia parameters for the gastro-resistance test.

For the tablet, active ingredients were chosen in a bioavailable form, although the most readily available active ingredients are never 100% absorbed. To account for this probable incomplete bioavailability, the dose of each active ingredient was increased by 20% compared to the previously described calculation, hypothesizing an absorption loss of this amount for each one.

This led to defining a dose for each active ingredient to be included in the tablet to be subjected to a clinical study (Table 1).

**Table 1. Composition of active ingredients in the final tablet.**

| **Active ingredient** | **Conc. in vitro** | **Hypothetical dose** | **Dose +20%** | **Final tablet** |
|---|---|---|---|---|
| Curcuma d.e. in bioavailable form tit. 20% curcuminoids | 4 µg/mL | 20 mg | 24 mg | Curcuma extract 24 mg (providing 4.8 mg curcuminoids) |
| Vitamin D3 powder 100,000 IU/g (equal to 0.25%) in water-dispersible form | 2.5 µg/mL | 12.5 mg | 15 mg | Cholecalciferol 100000 IU/g 15 mg of which Vitamin D3 37.5 mcg |
| Starting material comprising CoQ10 in bioavailable form tit. 25% | 10 µg/mL | 50 mg | 60 mg | Starting material 60 mg, of which Coenzyme Q10 15 mg |
| Vitamin B1 (Thiamine hydrochloride) | 4 µg/mL | 20 mg | 24 mg | Thiamine hydrochloride 24 mg of which Vitamin B1 19.4 mg |
| Black Garlic (Black garlic d.e. tit. 0.1% S-allyl-cysteine) | 15 µg/mL | 75 mg | 90 mg | Black garlic d.e. 90 mg (providing S-allyl-cysteine 0.09 mg) |

### 2.5. Effect of the oral supplement on endothelial function and arterial stiffness

In a clinical study conducted on 10 healthy subjects, the effect of the supplement, generated thanks to the *in vitro* studies, taken orally for 4 weeks using the EndoPat instrument was evaluated (Figure 5).

At the start of the study, 6 of the 10 subjects had a reactive hyperemia index (RHI) below the normality threshold (ln 0.51), indicating endothelial dysfunction.

After 4 weeks of treatment, no general improvement was observed in the parameters of all participants (Figure 6B).

However, focusing on the 6 subjects with confirmed endothelial dysfunction, the supplement showed a significantly positive effect (Figure 6C) and 50% of these subjects reached an endothelial function above the normality threshold in just 28 days. In addition, the arterial stiffness augmentation index decreased significantly in all treated subjects (Figures 6D-E), suggesting an improvement in overall vascular health. Hepatic safety was assessed by measuring plasma levels of alanine aminotransferase (ALT), which showed no variations throughout the treatment (Figure 7A).

Finally, systemic oxidative stress markers, such as advanced oxidation protein products (AOPP), malondialdehyde (MDA), and the activity of superoxide dismutase (SOD), were examined.

The levels of AOPP and MDA did not show variations during the entire treatment period (Figures 7B-C), while the activity of SOD, which represents a part of the human body's antioxidant capacity, showed a significant increase at the end of the treatment (Figure 7D).

These results indicate that the supplement is able to improve overall vascular health and has further specific benefits for individuals with pre-existing endothelial dysfunction.

### BIBLIOGRAPHY

1. Myasoedova, V.A., et al., Aortic Valve Sclerosis as an Important Predictor of Long-Term Mortality in Patients With Carotid Atheromatous Plaque Requiring Carotid 20 Endarterectomy. Front Cardiovasc Med, 2021. 8: p. 653991.
2. Myasoedova, V.A., et al., Aortic Valve Sclerosis in High-Risk Coronary Artery Disease Patients. Front Cardiovasc Med, 2021. 8: p. 711899.
3. Poggio, P., et al., Aortic Valve Sclerosis Adds to Prediction of Short-Term Mortality in Patients with Documented Coronary Atherosclerosis. J Clin Med, 2019. 8(8).
4. Otto, C.M., et al., Association of aortic-valve sclerosis with cardiovascular mortality and morbidity in the elderly. N Engl J Med, 1999. 341(3): p. 142-7.
5. Valerio, V., et al., Impact of Oxidative Stress and Protein S-Glutathionylation in Aortic Valve Sclerosis Patients with Overt Atherosclerosis. J Clin Med, 2019. 8(4).
6. Valerio, V., et al., Enduring Reactive Oxygen Species Emission Causes Aberrant Protein S-Glutathionylation Transitioning Human Aortic Valve Cells from a Sclerotic to a Stenotic Phenotype. Antioxid Redox Signal, 2022. 37(13-15): p. 1051-1071.
7. Hansson, G.K., Inflammatory mechanisms in atherosclerosis. J Thromb Haemost, 2009. 7 Suppl 1: p. 328-31.
8. Shu, L., et al., Oxidative stress and valvular endothelial cells in aortic valve calcification. Biomed Pharmacother, 2023. 163: p. 114775.
9. Ramaraj, R. and V.L. Sorrell, Degenerative aortic stenosis. BMJ, 2008. 336(7643): p. 550-5.
10. Rubanyi, G.M., The role of endothelium in cardiovascular homeostasis and diseases. J Cardiovasc Pharmacol, 1993. 22 Suppl 4: p. S1-14.
11. Sun, H.J., et al., Role of Endothelial Dysfunction in Cardiovascular Diseases: The Link Between Inflammation and Hydrogen Sulfide. Front Pharmacol, 2019. 10: p. 1568.
12. Godo, S. and H. Shimokawa, Endothelial Functions. Arterioscler Thromb Vasc Biol, 2017. 37(9): p. e108-e114.
13. Jeremias, A., et al., Effects of N-acetyl-cysteine on endothelial function and inflammation in patients with type 2 diabetes mellitus. Heart Int, 2009. 4(1): p. e7.
14. Pujadas, G., et al., Genetic disruption of the Gipr in Apoe(-/-) mice promotes atherosclerosis. Mol Metab, 2022. 65: p. 101586.
15. Mori, Y., et al., Glucose-Dependent Insulinotropic Polypeptide Suppresses Peripheral Arterial Remodeling in Male Mice. Endocrinology, 2018. 159(7): p. 2717-20 2732.
16. Mahootchi, E., et al., GADL1 is a multifunctional decarboxylase with tissue-specific roles in beta-alanine and carnosine production. Sci Adv, 2020. 6(29): p. eabb3713.
17. Kulig, P., et al., IL-12 protects from psoriasiform skin inflammation. Nat Commun, 25 2016. 7: p. 13466.
18. Hildenbrand, K., et al., Human interleukin-12alpha and EBI3 are cytokines with anti-inflammatory functions. Sci Adv, 2023. 9(43): p. eadg6874.
19. Dobin, A., et al., STAR: ultrafast universal RNA-seq aligner. Bioinformatics, 2013. 29(1): p. 15-21.
20. Liao, Y., G.K. Smyth, and W. Shi, featureCounts: an efficient general purpose program for assigning sequence reads to genomic features. Bioinformatics, 2014. 30(7): p. 923-30.
21. Chiesa, M., G.I. Colombo, and L. Piacentini, DaMiRseq-an R/Bioconductor 5 package for data mining of RNA-Seq data: normalization, feature selection and classification. Bioinformatics, 2018. 34(8): p. 1416-1418.

## Claims

1. An association of active ingredients comprising
- a dry extract of Curcuma,
- a dry extract of fermented black garlic,
- a coenzyme Q10,
- at least one vitamin selected from the group consisting of: at least one B-group vitamin; vitamin D; and combinations of the foregoing,
for use in the treatment and/or prevention of the dysfunction of the vascular and/or valvular endothelium of the cardiocirculatory system.

2. The association for use according to claim 1, wherein the dysfunction of the vascular and/or valvular endothelium manifests concomitantly with or is caused by a pathological state selected from the group consisting of: an inflammation of the endothelium; an increase in the rigidity of the vascular and/or valvular tone with respect to the physiological condition; a damage to the endothelium; an increase in reactive oxygen species with respect to the physiological condition; a vascular and/or valvular calcification; and mixtures of the foregoing.

3. The association for use according to claim 1 or 2, wherein the restoration of the physiological function of the vascular and/or valvular endothelium is subordinate to a dysfunction of the vascular and/or valvular endothelium that manifests concomitantly with cardiovascular pathologies selected from the group consisting of: an aortic valve sclerosis; an aortic stenosis; a coronary artery disease; and mixtures of the foregoing.

4. The association for use according to any one of claims 1 to 3, wherein the weight ratio between the amount of the coenzyme Q10 and the amount of the dry extract of Curcuma is comprised between 1:1.5 and 1:5.

5. The association for use according to any one of claims 1 to 4, wherein the weight ratio between the amount of the dry extract of black garlic and the amount of the dry extract of Curcuma is comprised between 1:2 and 1:7.

6. The association for use according to any one of claims 1 to 5, wherein the weight ratio between the amount of the dry extract of black garlic and the amount of the coenzyme Q10 is comprised between 1:1.2 and 1:4.

7. The association for use according to any one of claims 1 to 6, wherein the weight ratio between the sum of the amounts of dry extract of Curcuma, coenzyme Q10 and dry extract of black garlic and the amount of the at least one vitamin is comprised between 1:2 and 1:7.

8. The association for use according to any one of claims 1 to 7, wherein the at least one vitamin is a combination of thiamine and cholecalciferol.

9. The association for use according to any one of claims 1 to 8, wherein the dry extract of Curcuma comprises curcuminoids in an amount comprised between 10% and 50% by weight based on the total weight of the extract.

10. An oral formulation comprising the association for use according to any one of claims 1 to 9, in combination with suitable excipients and/or diluents.

11. The oral formulation according to claim 10, in the form of a food supplement, a nutraceutical, a medicinal specialty for human or animal use.
